# EUROPEAN PATENT SPECIFICATION

(11) **EP 2 093 217 B1**
(45) Date of publication and mention of the grant of the patent: **12.03.2014**
(21) Application number: 09160600.4
(22) Date of filing: 27.01.2006
(51) Int. Cl.: C07D 215/22, A61K 31/496, A61P 25/18

(54) **Process for preparing Form X of aripiprazole**
Verfahren zur Herstellung von Form X von Aripiprazol
Procédé de préparation de Forme X d'aripiprazole

(30) Priority: 27.01.2005 EP 05001638
(43) Date of publication of application: 26.08.2009
(62) Divisional of application: 06706450.1
(73) Proprietor: Sandoz AG, 4056 Basel (CH)
(72) Inventor: Wieser, Josef, 6250 Kundl (AT); Lengauer, Hannes, 6250 Kundl (AT); Braun, Doris, 6850 Dornbirn (AT); Griesser, Ulrich J., 6094 Axams (AT); Tessadri, Richard, 6020 Innsbruck (AT)
(74) Representative: Wichmann, Hendrik

(56) References cited:
- WO-A-2004/099152
- WO-A-2005/009990
- WO-A-2006/030446
- US-A1- 2004 058 935
- Satoshi Aoki et al.: "CR 119 Study on Crystal Transformation of ARIPIPRAZOL" In: "The Fourth Japan-Korea Symposium on Separation Technology", 1996

## Description

### FIELD OF THE INVENTION

The present invention relates to polymorphic forms of aripiprazole.

### BACKGROUND OF THE INVENTION

Aripiprazole, 7-[4-[4-(2,3-dichlorophenyl)-1-piperazinyl]butoxy]-3,4-dihydro-2(1H)-quinolinone, is an antipsychotic drug useful in the treatment of schizophrenia (Merck Index, monograph number 00791, CAS registry number 129722-12-9).
Synthesis and isolation of aripiprazole are described in EP 367141 B1 and US 5006528. Additional crystalline anhydrous or hydrous forms are disclosed in WO 03/026659 (conventional hydrate, hydrate A, conventional anhydrate, anhydrate B, anhydrate C, anhydrate D, anhydrate E, anhydrate F, anhydrate G).

The report of Aoki et al. in "The Fourth Japan-Korea Symposium on Separation Technology" October 6-8 1996, Waseda University International Conference Center, Tokyo, Japan, CR.119, describes two types of anhydrous aripiprazole crystals, Type I and Type II. The Type II crystals were prepared by conversion of Type I crystals by heating at 130°C-140°C for 15 hours.

Nevertheless, there remains a need for polymorphic forms of aripiprazole which have properties suitable for pharmaceutical processing on a commercial scale. The present invention satisfies these needs by providing polymorphic forms of aripiprazole.

### SUMMARY OF THE INVENTION

The present invention relates to processes for preparing form X of aripiprazole characterized by an X-ray powder diffraction pattern with peaks at 10.0, 11.6, 15.7, 16.3, 18.5, 20.4, 21.8, 22.2 and 23.3 degrees 2θ.

Further disclosed is aripiprazole ethanol hemisolvate characterized by an X-ray powder diffraction pattern with peaks at 17.4, 18.1, 19.6, 23.3 and 27.9 degrees 2θ.

Also disclosed is aripiprazole methanol solvate characterized by an X-ray powder diffraction pattern with peaks at 11.5, 17.3, 18.5, 19.8, 23.1, 24.4 and 26.9 degrees 2θ.

The present invention provides a process for preparing form X of aripiprazole, comprising the steps of:
a) dissolving aripiprazole in 2-propanol by heating
b) slowly cooling the solution to room temperature or below to effect crystallization
c) optionally isolating crystalline form X of aripiprazole.

The present invention also provides a process for preparing form X of aripiprazole characterized in that a suspension of aripiprazole in a solvent is seeded with crystals of form X and the suspension is stirred at a suitable temperature in order to effect transformation of the used form of aripiprazole into form X, wherein the solvent is 2-propanol, 1-butanol, or 1-propanol.

A process for preparing aripiprazole ethanol hemisolvate, comprises the steps of:
a) dissolving aripiprazole in ethanol by heating
b) slowly cooling the solution to room temperature or below to effect crystallization
c) isolating the crystalline ethanol hemisolvate
d) drying the ethanol hemisolvate at a temperature below about 60°C.

A further process for preparing aripiprazole ethanol hemisolvate, comprises the steps of:
a) dissolving aripiprazole in a solvent in which aripiprazole is soluble at room temperature
b) diluting the solution with ethanol
c) isolating the crystalline ethanol hemisolvate
d) drying the ethanol hemisolvate at a temperature below about 60°C.

A further process for preparing aripiprazole ethanol hemisolvate is characterized in that a suspension of aripiprazole in ethanol is stirred at a suitable temperature in order to effect transformation of the used form of aripiprazole into aripiprazole ethanol hemisolvate.

A process for preparing aripiprazole methanol solvate, comprises the steps of:
a) dissolving aripiprazole in methanol by heating
b) slowly cooling the solution to room temperature or below to effect crystallization
c) isolating the crystalline methanol solvate
d) drying the methanol solvate at a temperature below about 60°C.

A further process for preparing aripiprazole methanol solvate, comprises the steps of:
a) dissolving aripiprazole in a solvent in which aripiprazole is soluble at room temperature
b) diluting the solution with methanol
c) isolating the crystalline methanol solvate
d) drying the methanol solvate at a temperature below about 60°C.

A further process for preparing aripiprazole methanol solvate is characterized in that a suspension of aripiprazole in methanol is stirred at a suitable temperature in order to effect transformation of the used form of aripiprazole into aripiprazole methanol solvate.

Other objects, features, advantages and aspects of the present invention will become apparent to those of skill from the following description. It should be understood, however, that the description and the following specific examples, while indicating preferred embodiments of the invention, are given by way of illustration only.

### BRIEF DESCRIPTION OF THE DRAWINGS

Figure 1: Infrared spectrum of form X of aripiprazole.
Figure 2: Infrared spectrum of aripiprazole ethanol hemisolvate.
Figure 3: Infrared spectrum of aripiprazole methanol solvate.
Figure 4: X-ray powder diffraction pattern of form X of aripiprazole.
Figure 5: X-ray powder diffraction pattern of aripiprazole ethanol hemisolvate.
Figure 6: X-ray powder diffraction pattern of aripiprazole methanol solvate.
Figure 7: Thermogravimetric and differential scanning calorimetric curve of form X of aripiprazole.
Figure 8: Thermogravimetric and differential scanning calorimetric curve of aripiprazole ethanol hemisolvate.
Figure 9: Thermogravimetric and differential scanning calorimetric curve of aripiprazole methanol solvate.
Figure 10: Moisture sorption isotherm of form X of aripiprazole.

### DETAILED DESCRIPTION OF THE INVENTION

As used herein the term "crude" refers to crystals of aripiprazole that have not been washed and/or recrystallized to remove impurities that may be present.
As used herein the term "crystalline" refers to crystals of aripiprazole that have been washed and recrystallized to remove impurities.
As used herein the term "amorphous" relates to solid material which lacks a regular crystalline structure.
The term "room temperature" as used herein indicates that the applied temperature is not critical and that no exact temperature value has to be kept. Usually, "room temperature" is understood to mean temperatures of about 15°C to about 25°C (see e.g. EU Pharmacopeia 5.0, page 6).

The polymorphs of aripiprazole have distinct physical properties and may be characterized e.g. by a typical X-ray powder diffraction pattern, infrared spectrum or a characteristic thermogravimetric (TGA) and differential scanning calorimetric (DSC) curve. Each of these characteristics on its own is sufficient to unambiguously define and identify the polymorphs but they also may be combined with each other.

The present invention relates to processes for preparing form X of aripiprazole characterized by an X-ray powder diffraction pattern with peaks at 10.0, 11.6, 15.7, 16.3, 18.5, 20.4, 21.8, 22.2 and 23.3 degrees 2θ (2-theta).

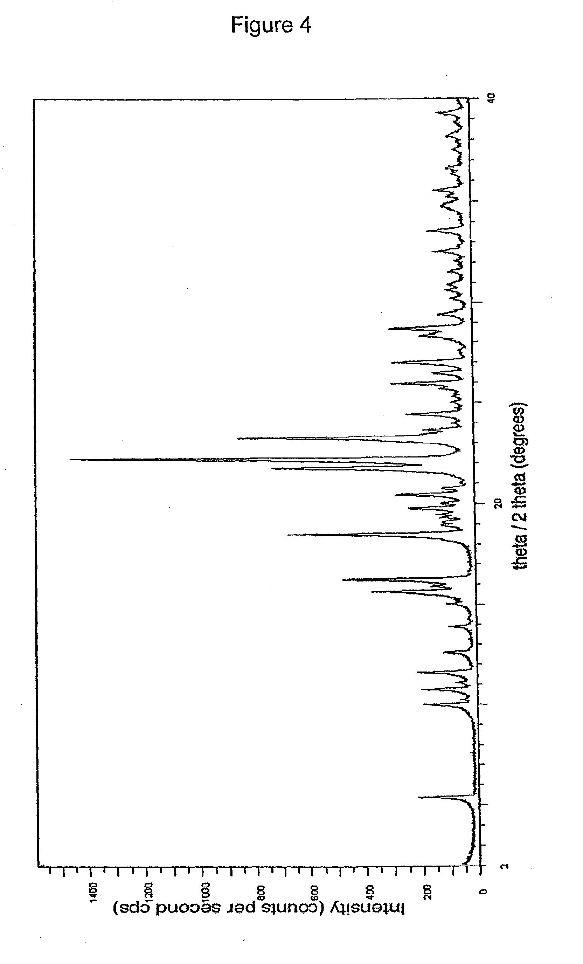

A characteristic X-ray powder diffraction pattern of form X of aripiprazole is shown in Figure 4 and some characteristic peaks are listed in Table 1. Accordingly, in a preferred embodiment, the present invention relates to processes for preparing form X of aripiprazole characterized by an X-ray powder diffraction pattern substantially in accordance with Table 1 and Figure 4.

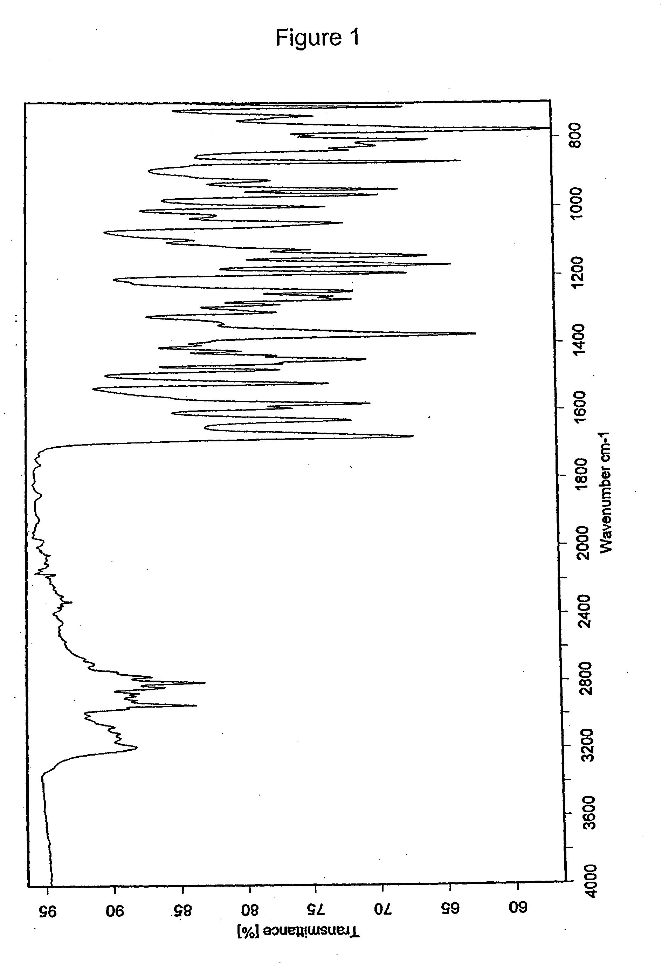

Form X of aripiprazole may be also characterized by a typical infrared spectrum as shown in Figure 1. Accordingly, in a further preferred embodiment, the present invention relates to processes for preparing form X of aripiprazole characterized by an infrared spectrum substantially in accordance with Figure 1. Characteristic bands are present at 2939, 2803, 1677, 1374, 1191, 1168, 1045, 868, 822 and 736 cm⁻¹.

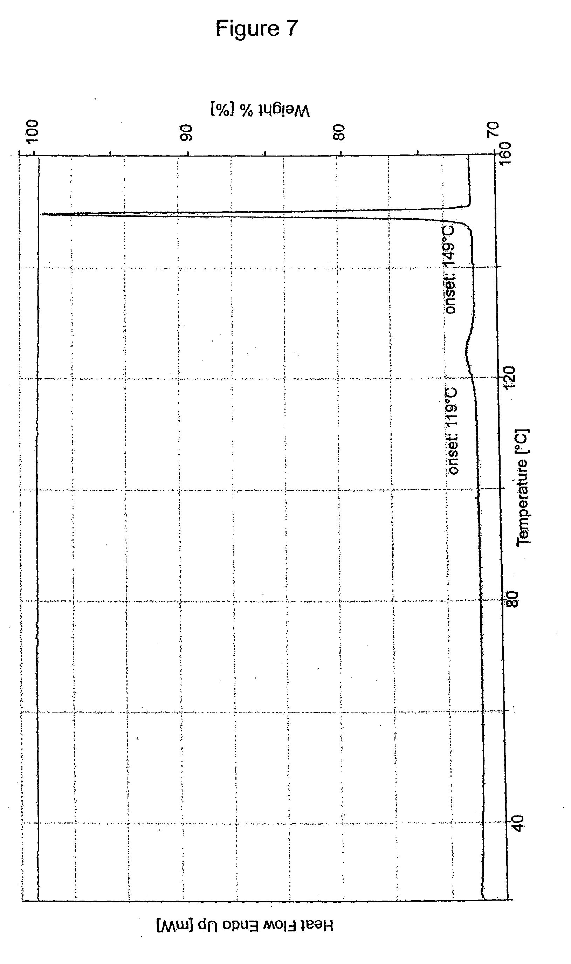

In addition, form X of aripiprazole shows a typical DSC curve at a heating rate of of 5°K/min and practically no mass loss in the TGA analysis. Typical thermograms of form X of aripiprazole are shown in Figure 7. It can be seen that the DSC curve of form X shows two specific endothermic peaks with onset temperatures of about 119°C and 149°C.

Accordingly, in a further preferred embodiment, the present invention relates to processes for preparing form X of aripiprazole characterized by two endothermic peaks with onset temperatures of about 119°C and 149°C at a heating rate of 5°K/min in differential scanning calorimetry.

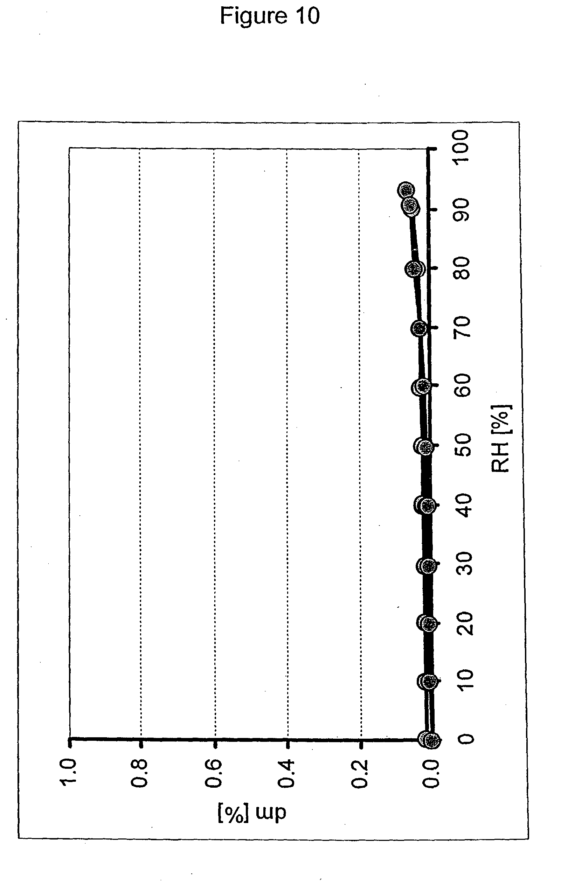

Form X of aripiprazole is a anhydrous form, hereinafter also referred to as "form X", which is particularly stable and hence is suitable for bulk preparation and handling. Form X of aripiprazole has been found to be of low hygroscopicity and does not substantially convert into a hydrated form of aripiprazole. This can be seen from the moisture sorption isotherm of form X which is shown in Figure 10.

Further disclosed is aripiprazole ethanol hemisolvate characterized by an X-ray powder diffraction pattern with peaks at 17.4, 18.1, 19.6, 23.3 and 27.9 degrees 2θ.

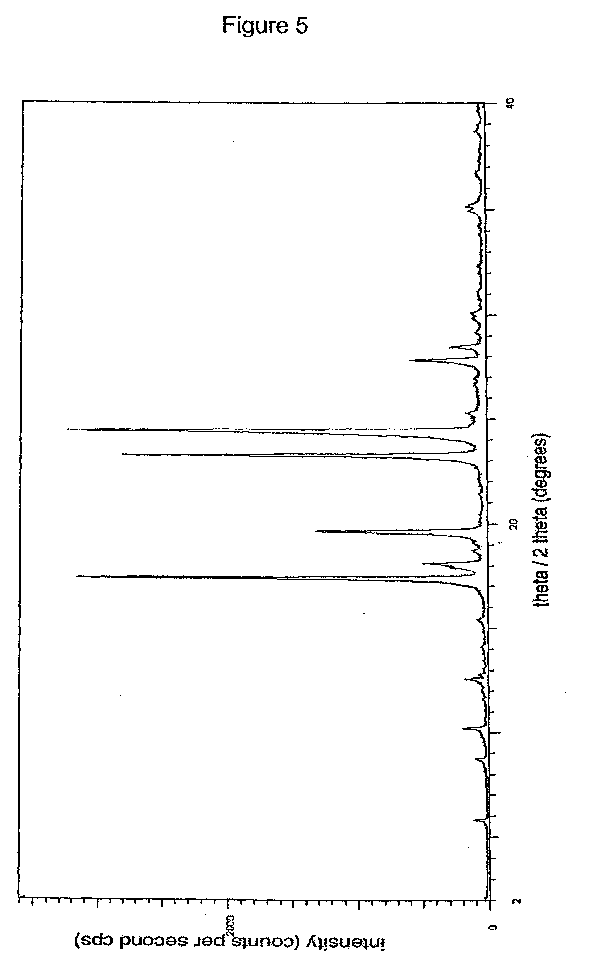

A characteristic X-ray powder diffraction pattern of aripiprazole ethanol hemisolvate is shown in Figure 5 and some characteristic peaks are listed in Table 2.

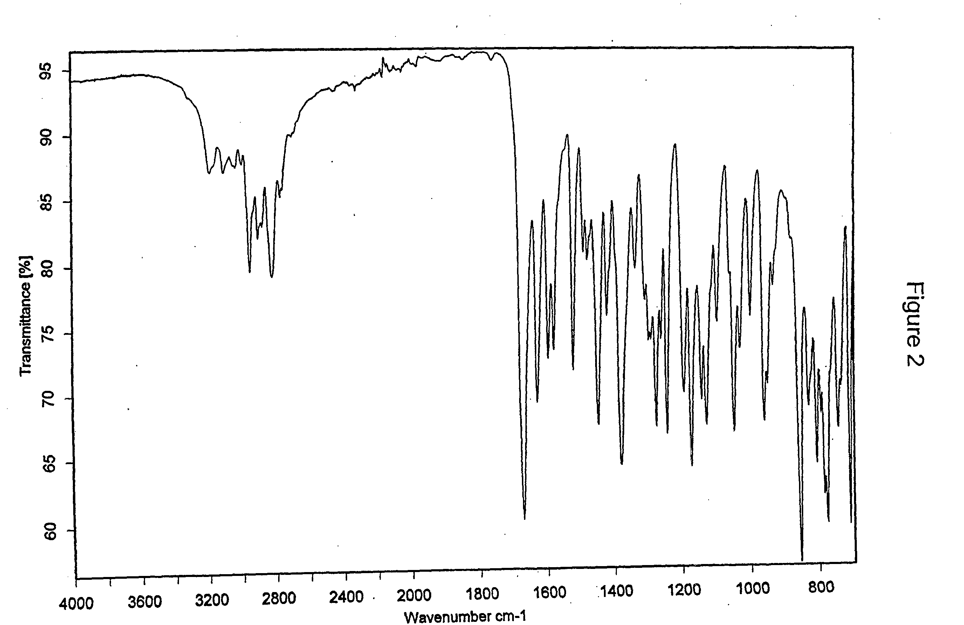

Aripiprazole ethanol hemisolvate may be also characterized by a typical infrared spectrum as shown in Figure 2. Characteristic bands are present at 2949, 2816, 1670, 1378, 1192, 1171, 1047, 854, 830 and 746 cm⁻¹.

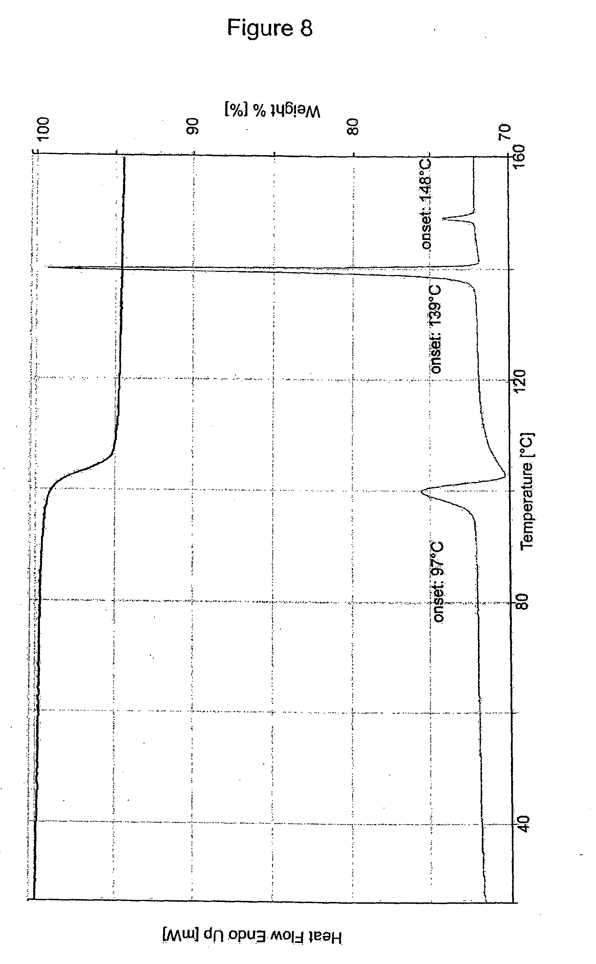

In addition, aripiprazole ethanol hemisolvate shows a typical step in the TGA curve and a characteristic DSC curve at a heating rate of of 5°K/min. Typical thermograms of aripiprazole ethanol hemisolvate are shown in Figure 8. It can be seen that the DSC curve of aripiprazole ethanol hemisolvate (sample pan with pinhole) shows an endothermic peak with an onset temperature of about 97°C followed by an exothermic event and two endothermic peaks with onset temperatures of about 139°C and 148°C.

Aripiprazole methanol solvate is characterized by an X-ray powder diffraction pattern with peaks at 11.5, 17.3, 18.5, 19.8, 23.1, 24.4 and 26.9 degrees 2θ.

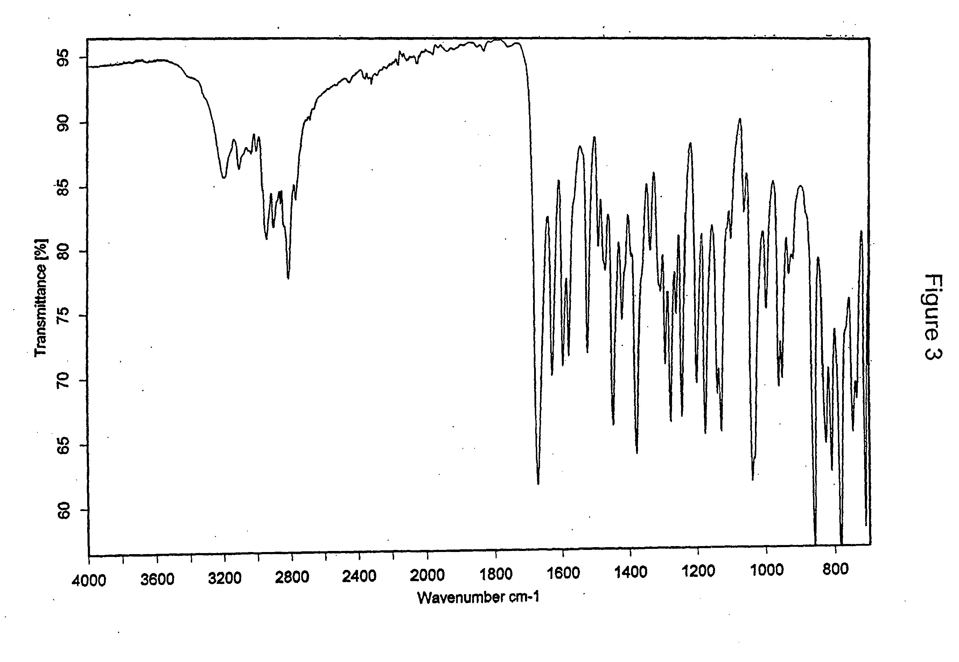

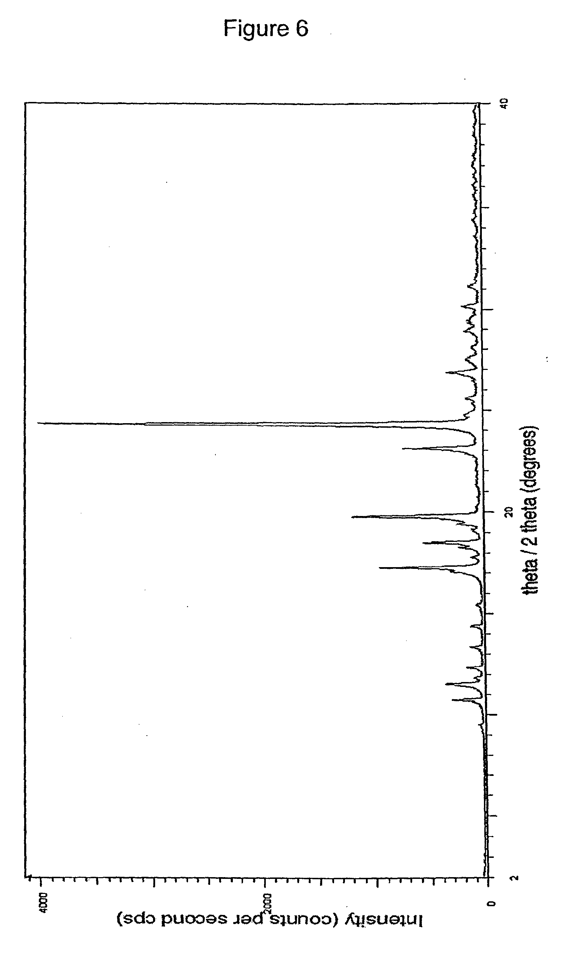

A characteristic X-ray powder diffraction pattern of aripiprazole methanol solvate is shown in Figure 6 and some characteristic peaks are listed in Table 3. Aripiprazole methanol solvate may be also characterized by a typical infrared spectrum as shown in Figure 3. Characteristic bands are present at 2942, 2814, 1671, 1377, 1199, 1174, 1037, 857, 824 and 746 cm⁻¹.

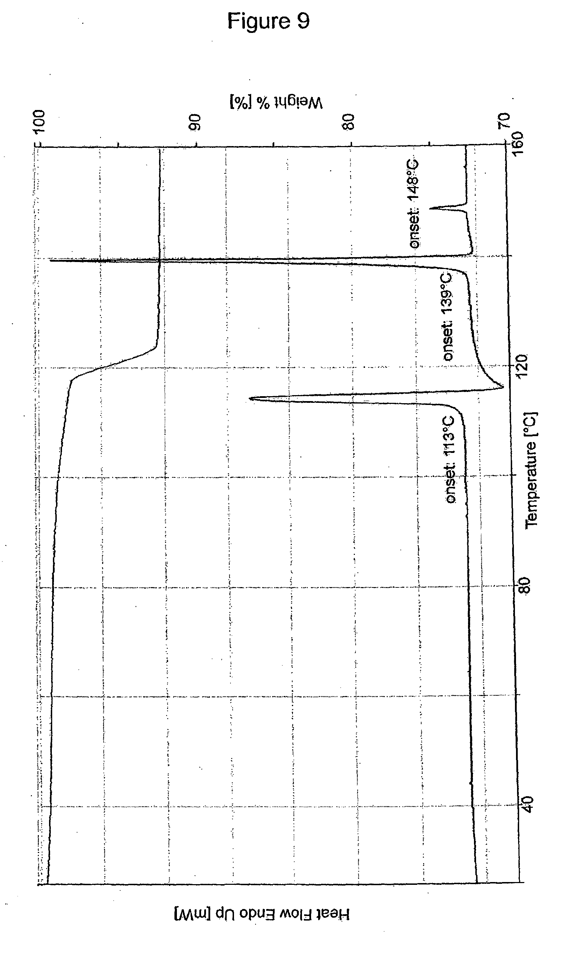

In addition, aripiprazole methanol solvate shows a typical step in the TGA curve and a characteristic DSC curve at a heating rate of of 5°K/min. Typical thermograms of aripiprazole methanol solvate are shown in Figure 9. It can be seen that the DSC curve of aripiprazole methanol solvate (sample pan with pinhole) shows an endothermic peak with an onset temperature of about 113°C followed by an exothermic event and two endothermic peaks with onset temperatures of about 139°C and 148°C.

As said before, each of the above mentioned data alone are sufficient to characterize each of the polymorphs of aripiprazole.

In one embodiment, the present invention provides a first process for preparing form X of aripiprazole, comprising the steps of:
a) dissolving aripiprazole in 2-propanol by heating
b) slowly cooling the solution to room temperature or below to effect crystallization
c) optionally isolating crystalline form X of aripiprazole.

For preparing form X of aripiprazole according to the above first process, any other form of aripiprazole may be used, e.g. the forms disclosed in WO 03/026659 (conventional hydrate, hydrate A, conventional anhydride, anhydrate B, anhydrate C, anhydrate D, anhydrate E, anhydrate F, anhydrate G). In addition, also non-crystalline forms of aripiprazole such as the amorphous form and forms of low crystalline order or crude aripiprazole may be used.

In particular, the other novel forms of aripiprazole of the present invention described above, i.e. aripiprazole ethanol hemisolvate and aripiprazole methanol solvate or an aripiprazole hydrate may be used to prepare form X of aripiprazole. Of course, also mixtures of two or more different forms of aripiprazole may be used. Therefore, in a preferred embodiment, the form used in the above first process in step a) is aripiprazole ethanol hemisolvate and/or aripiprazole methanol solvate and/or aripiprazole hydrate.

According to the above first process the solvent is 2-propanol.

The heating temperature applied in order to dissolve aripiprazole depends on the boiling point of the solvent used but usually will be in the range of 50°C to 100°C. A preferred temperature is the boiling temperature of the solvent. If 2-propanol is used, the aripiprazole is dissolved preferably in a concentration of about 20 g to about 40 g per liter 2-propanol by heating, most preferably in a concentration of about 30 g to about 35 g aripiprazole per liter 2-propanol.

The crystallization step b) of the above first process may be facilitated by adding seed crystals of form X of aripiprazole. Accordingly, in a preferred embodiment, in the above first process in step b) seed crystals of form X of aripiprazole are added. In a preferred embodiment seeds are added to a solution of aripiprazole in 2-propanol at a temperature of between approximately 80°C to 40°C , preferably at a temperature of about 70°C to 50°C , preferably at a temperature of about 70°C to 60°C and the suspension is then cooled slowly to a temperature of about 60°C to 40°C preferably to a temperature of about 50°C to 40°C in a first step followed by further cooling of the suspension to about 20°C to 0°C , preferably 10°C to 0°C in a second step. Optionally the suspension is heated again to a temperature of 40°C to 60°C, preferably to a temperature of about 50°C to 60°C and cooled again as described above.

In another embodiment, the present invention provides a second process for preparing form X of aripiprazole characterized in that a suspension of aripiprazole in a solvent as defined in the claims is seeded with crystals of form X and the suspension is stirred at a suitable temperature in order to effect transformation of the used form of aripiprazole into form X.

Just as for the first process described above also for this second process for preparing form X of aripiprazole any other form of aripiprazole, i.e. any crystalline unsolvated or solvated form, non- crystalline or amorphous form or crude aripiprazole may be used. Preferably, the other novel forms of aripiprazole of the present invention, i.e. aripiprazole ethanol hemisolvate and aripiprazole methanol solvate or an aripiprazole hydrate may be used.

However, this second process is based on the solution mediated transformation of any form of aripiprazole which is thermodynamically less stable under given conditions than form X. Therefore, the transformation into form X is a thermodynamically controlled process due to the fact that form X has the lowest Gibbs free energy at temperatures at and below about 60°C. Consequently, all known solid crystalline or non-crystalline forms, of aripiprazole may be used in the present process.

According to the above second process the solvent is selected from 2-propanol, 1-butanol or 1-propanol.

The temperature at which the suspension is stirred in order to effect transformation of the suspended form of aripiprazole into form X depends on the form of aripiprazole and the solvent used. Room temperature or an elevated temperature may be applied but usually it will be in the range of 10°C to 60°C. However, it is crucial that solvent and temperature are chosen such that the used form of aripiprazole remains in the condition of a suspension and does not become dissolved. It is well within the general knowledge of a person skilled in the art to determine a suitable solvent and temperature accordingly.
In a preferred embodiment the solvent is 2-propanol and the preferred temperature range is about 10°C to about 80°C, preferably at a temperature of about 60°C to about 40°C, most preferable at a temperature of about 60°C to about 50°C for a time to form form X of aripiprazole.. Aripiprazole is then isolated after cooling the solution. A time sufficient for the formation of form X varies from a view minutes , e.g. 15 to 30 min to several days, e.g. 2 to 6 days. Optionally in intervals the suspension may be cooled to a lower temperature, e.g. to a temperature to about ambient temperature and the suspension is then reheated to the desired temperature.

A process for preparing aripiprazole ethanol hemisolvate, comprises the steps of:
a) dissolving aripiprazole in ethanol by heating
b) slowly cooling the solution to room temperature or below to effect crystallization
c) isolating the crystalline ethanol hemisolvate
d) drying the ethanol hemisolvate at a temperature below about 60°C.

Just as for the processes for preparing form X of aripiprazole described above also for this first process for preparing aripiprazole ethanol hemisolvate any other form of aripiprazole, i.e. any crystalline unsolvated or solvated form, any non-crystalline or amorphous form and crude aripiprazole may be used.

The heating temperature applied in order to dissolve aripiprazole depends on the boiling point of the solvent used but usually will be in the range of 50°C to 100°C. A preferred temperature is the boiling temperature of the solvent.

Drying the ethanol hemisolvate at a temperature below about 60°C may be performed in different ways under different suitable conditions. Drying time and drying temperature are inversely related, so that for example the drying time will be longer the lower the drying temperature, and shorter the higher the drying temperature. Particularly suitable and preferred are air drying at room temperature, drying under vacuum at room temperature over night or drying at 50°C for 2 hours.

Alternatively, instead of using pure ethanol as solvent for preparing the ethanol hemisolvate, aripiprazole may be dissolved in a solvent in which aripiprazole is soluble at room temperature such as e.g. methylene choride, tetrahydrofuran or amides like e.g. dimethylformamide, dimethylacetamide or N-methylpyrrolidone. A suitable solvent is a solvent which allows dissolving one gram aripiprazole in a volume of approximately 10 ml solvent or less at room temperature. Due to the good solubility only a little quantity of the solvent is needed. The concentrated solution is then diluted with ethanol resulting in the crystallization of aripiprazole ethanol hemisolvate.

This process provides high yields of aripiprazole ethanol hemisolvate and also represents a practical method for purification of crude aripiprazole, because most of the impurities of crude aripiprazole are more soluble than aripiprazole ethanol hemisolvate and remain in solution.

A second process for preparing aripiprazole ethanol hemisolvate, comprises the steps of:
a) dissolving aripiprazole in a solvent in which aripiprazole is soluble at room temperature
b) diluting the solution with ethanol
c) isolating the crystalline ethanol hemisolvate
d) drying the ethanol hemisolvate at a temperature below about 60°C.

Preferably, the solvent used in the above second process for preparing aripiprazole ethanol hemisolvate is selected from methylene chloride, tetrahydrofuran, dimethylformamide, dimethylacetamide or N-methylpyrrolidone.

With respect to the aripiprazole form used in step a) and the performance of drying step d) the same applies as said above for the first process for preparing aripiprazole ethanol hemisolvate.

A third process for preparing aripiprazole ethanol hemisolvate is characterized in that a suspension of aripiprazole in ethanol is stirred at a suitable temperature in order to effect transformation of the used form of aripiprazole into aripiprazole ethanol hemisolvate.

With respect to the aripiprazole form used the same applies as said above for the first and second process for preparing aripiprazole ethanol hemisolvate.

The temperature at which the suspension is stirred in order to effect transformation of the suspended form of aripiprazole into aripiprazole ethanol hemisolvate may be room temperature or an elevated temperature. Usually, it will be in the range of 10°C to 40°C. However, it is crucial that the temperature is chosen such that the used form of aripiprazole remains in the condition of a suspension and does not become dissolved. It is well within the general knowledge of a person skilled in the art to determine a suitable temperature accordingly.

A first process for preparing aripiprazole methanol solvate comprises the steps of:
a) dissolving aripiprazole in methanol by heating
b) slowly cooling the solution to room temperature or below to effect crystallization
c) isolating the crystalline methanol solvate
d) drying the methanol solvate at a temperature below about 60°C.

Also for this first process for preparing aripiprazole methanol solvate any form of aripiprazole, i.e. any crystalline unsolvated or solvated form, any non-crystalline or amorphous form and crude aripiprazole may be used. Preferably, the other forms of aripiprazole of the present invention, i.e. form X of aripiprazole and aripiprazole ethanol hemisolvate or an aripiprazole hydrate may be used.

Drying the methanol solvate at a temperature below about 60°C may be performed in different ways under different suitable conditions. Drying time and drying temperature are inversely related, so that for example the drying time will be longer the lower the drying temperature, and shorter the higher the drying temperature. Particularly suitable and preferred are air drying at room temperature, drying under vacuum at room temperature over night or drying at 50°C for 2 hours.

Alternatively, instead of using pure methanol as solvent for preparing the methanol solvate, aripiprazole may be dissolved in a solvent in which aripiprazole is soluble at room temperature such as e.g. methylene choride, tetrahydrofuran or amides like e.g. dimethylformamide, dimethylacetamide or N-methylpyrrolidone. A suitable solvent is a solvent which allows dissolving one gram aripiprazole in a volume of approximately 10 millilitres solvent or less at room temperature. Due to the good solubility only a little quantity of the solvent is needed. The concentrated solution is then diluted with methanol resulting in the crystallization of aripiprazole methanol solvate.

This process provides high yields of aripiprazole methanol solvate and also represents a practical method for purification of crude aripiprazole, because most of the impurities of crude aripiprazole are more soluble than aripiprazole methanol solvate and remain in solution.

A second process for preparing aripiprazole methanol solvate, comprises the steps of:
a) dissolving aripiprazole in a solvent in which aripiprazole is soluble at room temperature
b) diluting the solution with methanol
c) isolating the crystalline methanol solvate
d) drying the methanol solvate at a temperature below about 60°C.

Preferably, the solvent used in the above second process for preparing aripiprazole methanol solvate is selected from methylene chloride, tetrahydrofuran, dimethylformamide, dimethylacetamide or N-methylpyrrolidone.

With respect to the aripiprazole form used in step a) and the performance of drying step d) the same applies as said above for the first process for preparing aripiprazole methanol solvate.

A third process for preparing aripiprazole methanol solvate is characterized in that a suspension of aripiprazole in methanol is stirred at a suitable temperature in order to effect transformation of the used form of aripiprazole into aripiprazole methanol solvate.

With respect to the aripiprazole form used the same applies as said above for the first and second process for preparing aripiprazole methanol solvate.

The temperature at which the suspension is stirred in order to effect transformation of the suspended form of aripiprazole into aripiprazole methanol solvate may be room temperature or an elevated temperature. Usually, it will be in the range of 10 °C to 40 °C. However, it is crucial that the temperature is chosen such that the used form of aripiprazole remains in the condition of a suspension and does not become dissolved. It is well within the general knowledge of a person skilled in the art to determine a suitable temperature accordingly.

The forms of aripiprazole disclosed herein may be used alone as antipsychotic drugs or in the form of a suitable pharmaceutical composition containing the novel form together with one or more suitable pharmaceutically acceptable carriers. The pharmaceutical compositions containing the forms of aripiprazole are prepared according to known processes.

The forms of aripiprazole disclosed herein are particularly useful for the treatment of schizophrenia.

A pharmaceutical composition comprises an effective amount of form X of aripiprazole or aripiprazole ethanol hemisolvate and a pharmaceutically acceptable carrier. The pharmaceutical composition may be prepared via a wet granulation process as known in the state of the art and as described for other crystalline and polymorphic forms in WO 03/026659. Another suitable preparation may be forming flash-melt tablets.

The invention is further described by reference to the following examples. These examples are provided for illustration purposes only and are not intended to be limiting the present invention in any way.

### EXAMPLES

The infrared spectra are recorded using a BRUKER FTIR-Tensor 27 with diamond ATR. The XRPD is recorded on a AXS-BRUKER X-ray powder diffractometer D-8 using the following acquisition conditions: tube anode: Cu; generator tension: 40 kV; generator current: 40 mA; start angle: 2.0°θ; end angle: 40.0°θ; step size: 0.01°θ; time per step: 2 seconds. Differential scanning calorimetry (DSC) is performed with a DSC 7 (Perkin-Elmer, Norwalk, Ct., USA) using the Pyris 2.0 software. Samples are weighed into 25 µl Al-Pans. Dry nitrogen is used as the purge gas (purge: 20 ml min⁻1)
Thermogravimetric analysis is performed with the Thermogravimetric-System TGA-7 using the Pyris-Software for Windows NT, (Perkin-Elmer, Norwalk, Ct., USA), 50 µL platin-pans, nitrogen purge gas (sample purge: 20 mL min⁻¹, balance purge: 40 mL min⁻¹).

### Example 1

25 g of aripiprazole Anhydrate Form A obtained as described in EP 367141 B1 are stirred in 250 ml isopropanol and heated at boiling until a clear solution is obtained. After cooling to room temperature under stirring the precipitated product is filtered and dried at room temperature under vacuum over night to give 24.12 g (96.5%) of the anhydrous polymorph referred to herein as form X.
The form X crystals of aripiprazole anhydride obtained above provide an infrared spectrum with peaks at 2939, 2803, 1677, 1374, 1191, 1168, 1045, 868, 822 and 736 cm⁻¹ (Figure 1). The XRPD pattern of form X of aripiprazole with characteristic XRPD angles, d-spacings and relative intensities is shown in Table 1 and in Figure 4.

**Table 1: X-Ray Powder Diffraction (XRPD) pattern of form X of aripiprazole. Values: Interplanar spacings (d, in Å, Angstroem), characteristic XRPD angles (in degrees 2-theta) and relative intensities (in %)**

| °Angle 2 theta | d-value (Å) | Rel. Intensity (%) |
|---|---|---|
| 5.3 | 16.56 | 7 |
| 10.0 | 8.88 | 22 |
| 10.7 | 8.24 | 7 |
| 11.1 | 7.99 | 8 |
| 11.6 | 7.64 | 22 |
| 12.6 | 7.04 | 10 |
| 15.7 | 5.66 | 23 |
| 16.3 | 5.45 | 41 |
| 18.5 | 4.79 | 21 |
| 18.9 | 4.69 | 8 |
| 19.8 | 4.48 | 14 |
| 20.0 | 4.43 | 12 |
| 20.4 | 4.34 | 20 |
| 21.8 | 4.08 | 39 |
| 22.2 | 4.00 | 100 |
| 23.3 | 3.81 | 24 |
| 24.4 | 3.64 | 9 |
| 26.0 | 3.42 | 13 |
| 27.0 | 3.29 | 9 |
| 28.8 | 3.10 | 13 |
| 33.6 | 2.66 | 6 |
| 35.3 | 2.54 | 7 |
| 35.6 | 2.52 | 6 |
| 39.4 | 2.29 | 6 |

Form X of aripiprazole shows a typical DSC curve at a heating rate of of 5°K/min and practically no mass loss in the TGA analysis. Typical thermograms of form X of aripiprazole are shown in Figure 7. It can be seen that form X shows two specific endothermic peaks with onset temperatures of about 119°C and 149°C.

### Example 2 (for comparison)

5 g of aripiprazole Anhydrate Form A obtained as described in EP 367141 B1 are stirred in 20 ml tetrahydrofuran and heated at boiling until a clear solution is obtained. After cooling to room temperature the suspension is stirred for additional three hours and then allowed to stand two days in a refrigerator. The precipitated product is filtered and dried at room temperature under vacuum over night to give 2.95 g (59%) of form X of the product.

### Example 3 (for comparison)

A suspension of 7 g of aripiprazole Anhydrate Form A obtained as described in reference example 1 of WO 03/026659 is stirred in 50 ml acetone and heated to reflux for 7.5 hours. The mixture is filtered and the insoluble solid dried in an desiccator at room temperature over night to yield 4.48 g (64%) of form X of the product.

### Example 4 (for comparison)

100 g crude aripiprazole prepared as described in EP 367141 B1 are suspended in 1500 ml absolute ethanol and then heated to reflux. The resulting solution is slowly cooled to room temperature to crystallize the product. After stirring at 0°C for one hour the precipitated product is filtered and dried at room temperature under vacuum for 17 hours to give 102.7 g (97,7%) of the ethanol hemisolvate of the product. The crystals are colorless plate form. The content of ethanol in the product is about 5,0 % by weight i.e. the compound is an ethanol hemisolvate containing half a mol of ethanol per mol aripiprazole.
The ethanol hemisolvate of aripiprazole obtained above provides an infrared spectrum with peaks at 2949, 2816, 1670, 1378, 1192, 1171, 1047, 854, 830 and 746 cm⁻¹ (Figure 2).
The XRPD pattern of aripiprazole ethanol hemisolvate with characteristic XRPD angles, d-spacings and relative intensities is shown in Table 2 and in Figure 5.

**Table 2: X-Ray Powder Diffraction (XRPD) pattern of aripiprazole ethanol hemisolvate. Values: Interplanar spacings (d, in Å, Angstroem), characteristic XRPD angles (in degrees 2-theta) and relative intensities (in %)**

| °Angle 2 theta | d-value (Å) | Rel. Intensity (%) |
|---|---|---|
| 5.8 | 15.28 | 3 |
| 8.6 | 10.22 | 2 |
| 10.2 | 8.70 | 4 |
| 12.5 | 7.05 | 4 |
| 15.4 | 5.75 | 2 |
| 16.7 | 5.31 | 3 |
| 17.4 | 5.09 | 55 |
| 18.1 | 4.90 | 8 |
| 18.7 | 4.74 | 3 |
| 19.6 | 4.52 | 22 |
| 23.3 | 3.82 | 36 |
| 24.5 | 3.64 | 100 |
| 25.3 | 3.52 | 3 |
| 27.9 | 3.20 | 9 |
| 28.5 | 3.13 | 3 |
| 35.0 | 2.56 | 3 |

Aripiprazole ethanol hemisolvate shows a typical endothermic curve in TGA and DSC analysis at a heating rate of of 5°K/min. A typical thermogram of aripiprazole ethanol hemisolvate is shown in Figure 8. It can be seen that aripiprazole ethanol hemisolvate shows an endothermic peak with an onset temperature of about 97°C followed by an exothermic event and two endothermic peaks with onset temperatures of about 139°C and 148.5°C.

### Example 5 (for comparison)

100 g crude aripiprazole prepared as described in EP 367141 B1 are suspended in 1500 ml absolute ethanol and then heated to reflux. The resulting solution is slowly cooled to room temperature to crystallize the product. After stirring at 0°C for one hour the precipitated product is filtered and dried at 50°C under vacuum for 2 hours.

### Example 6 (for comparison)

15 g of aripiprazole Anhydrate Form A obtained as described in EP 367141 B1 is added to 1600 ml methanol and the mixture is heated to reflux to get a clear solution. The solution is then slowly cooled to room temperature, stirred for another two hours and allowed to stand in a refrigerator for several days. The precipitated crystals are isolated by suction and dried at room temperature in vacuo to yield 15.2 g (93.7%) methanol solvate of the product. The crystals are colorless plate form. The content of methanol in the product is about 6,7% by weight i.e. the compound is a methanol monosolvate containing one mol of methanol per mol aripiprazole.
The methanol solvate of aripiprazole obtained above provides an infrared spectrum with peaks at 2942, 2814, 1671, 1377, 1199, 1174, 1037, 857, 824 and 746 cm⁻¹ (Figure 3).
The XRPD pattern of aripiprazole methanol solvate with characteristic XRPD angles, d-spacings and relative intensities is shown in Table 3 and in Figure 6.

**Table 3: X-Ray Powder Diffraction (XRPD) pattern of aripiprazole methanol solvate. Values: Interplanar spacings (d, in Å, Angstroem), characteristic XRPD angles (in degrees 2-theta) and relative intensities (in %)**

| °Angle 2 theta | d-value (Å) | Rel. Intensity (%) |
|---|---|---|
| 5.7 | 15.41 | <1 |
| 9.4 | 9.38 | 1 |
| 10.7 | 8.28 | 2 |
| 11.5 | 7.70 | 5 |
| 11.8 | 7.50 | 1 |
| 12.3 | 7.19 | 1 |
| 13.3 | 6.64 | 1 |
| 14.3 | 6.18 | 1 |
| 15.4 | 5.74 | 1 |
| 17.3 | 5.13 | 13 |
| 17.7 | 5.00 | 1 |
| 18.5 | 4.79 | 4 |
| 19.1 | 4.64 | 2 |
| 19.8 | 4.48 | 19 |
| 22.1 | 4.03 | 2 |
| 23.1 | 3.85 | 10 |
| 24.4 | 3.65 | 100 |
| 25.6 | 3.47 | 2 |
| 26.9 | 3.31 | 10 |
| 30.2 | 2.96 | 2 |
| 34.4 | 2.60 | 1 |

Aripiprazole methanol solvate shows a typical endothermic curve in TGA and DSC analysis at a heating rate of of 5°K/min. A typical thermogram of aripiprazole methanol solvate is shown in Figure 9. It can be seen that aripiprazole methanol solvate shows an endothermic peak with an onset temperature of about 113°C followed by an exothermic event and two endothermic peaks with onset temperatures of about 139°C and 148°C.

### Example 7 (for comparison)

1 g of crude aripiprazole Anhydrate Form A obtained as described in EP 367141 B1 is dissolved in 10 ml dichloromethane. The solution is diluted with 40 ml methanol and then allowed to stand in a refrigerator overnight. The precipitated crystals are isolated by suction and dried at room temperature in vacuo to yield 0.93 g (86.0%) methanol solvate of the product.

### Example 8

5 g of the ethanol hemisolvate of example 4 are recrystallized from 55 ml isopropanol using seed crystals obtained in example 1 above. The yield is 4.5 g (94.6%) of form X of aripiprazole.

### Example 9

Example 8 is repeated using 3 g of the methanol solvate obtained in example 5 above instead of the ethanol hemisolvate. The yield is 2.7 g (96.8%) of form X of aripiprazole.

### Example 10

Example 8 is repeated but instead of using the ethanol hemisolvate as starting material 5 g of the aripiprazole hydrate obtained as described in reference example 3 of WO 03/026659 is used. The yield is 4.5 g (93.6%) of form X of aripiprazole.

### Example 11

5 g of aripiprazole Anhydrate Form A obtained as described in EP 367141 B1 and 0.5 g of form X of aripiprazole are suspended in 55 ml isopropanol and heated at 50°C overnight and then stirred at room temperature for four days. The unsoluble part of the reaction mixture is collected by filtration and dried in vacuo at room temperature over night to give 4.6 g (92%) of form X of aripiprazole.

### Example 12 (for comparison)

5 g of aripiprazole Anhydrate Form A obtained as described in EP 367141 B1 are suspended in 50 ml ethanol and stirred at room temperature for three hours. The solid is filtered and dried at room temperature under vacuum for 17 hours to give 5.0 g (95,1%) of the ethanol hemisolvate of aripiprazole.

### Example 13

A suspension of 33 g of aripiprazole Anhydrate Form A obtained as described in EP 367141 B1 in 1000 ml of isopropanol is heated to reflux temperature. The resulting clear solution is cooled down to 65°C under stirring and then seeded with 0,3 g of aripiprazole form X. After further cooling down to 60°C a second portion of 0,3 g seeds of aripiprazole form X are added. The mixture is then slowly cooled down to 50°C in one hour and then from 50°C to 0°C in one hour. After stirring at 0°C for one hour, the obtained suspension is reheated to 50°C, stirred at this temperature for one or two hours, then again cooled to 0°C. After stirring for one hour the precipitated product is filtered and dried in vacuo at room temperature over night or at 60°C for 3 hours to yield 31,8 g (94,5%) of form X of aripiprazole

### Example 14

5 g aripiprazole hydrate obtained as described in example 22 are suspended in 80 ml isopropanol, seeded with 0,5 g aripiprazole form X and stirred at 50°C over night. The suspension is cooled to room temperature, filtered and dried at room temperature under vacuum for 17 hours to give 4,61 g (85,5%) aripiprazole form X .

### Example 15

Example 14 is repeated using 5 g of the ethanol hemisolvate as starting material instead of the hydrate. The yield is 4,51 g (84,3%) of form X of aripiprazole.

### Example 16

2 g aripiprazole methanol solvate obtained as described in example 6 are suspended in 30 ml isopropanol, seeded with 0,5 g aripiprazole form X and stirred at 50°C over night. The suspension is cooled to room temperature, filtered and dried at room temperature under vacuum for 17 hours to give 1,94g (77,2%) aripiprazole form X.

## Claims

1. Process for preparing form X of aripiprazole **characterized by** an X-ray powder diffraction pattern with peaks at 10.0, 11.6, 15.7, 16.3, 18.5, 20.4, 21.8, 22.2 and 23.3 degrees 2θ (2-theta), comprising the steps of:
a) dissolving aripiprazole in 2-propanol by eating
b) slowly cooling the solution to room temperature or below to effect crystallization
c) optionally isolating crystalline form X of aripiprazole.

2. Process according to claim 1, wherein the form used in step a) is aripiprazole ethanol hemisolvate and/or aripiprazole methanol solvate and/or aripiprazole hydrate.

3. Process for preparing form X of aripiprazole according to claim 1 or claim 2,
wherein in step b) seed crystals of form X of aripiprazole are added.

4. Process according to claim 3, wherein the solvent is 2-propanol and the preferred temperature range is about 40 °C to about 70 °C.

5. Process for preparing form X of aripiprazole **characterized by** an X-ray powder diffraction pattern with peaks at 10.0, 11.6, 15.7, 16.3, 18.5, 20.4, 21.8, 22.2 and 23.3 degrees 2θ (2-theta), **characterized in that** a suspension of aripiprazole in a solvent selected from 2-propanol, 1-butanol or 1-propanol is seeded with crystals of form X and the suspension is stirred at a suitable temperature in order to effect transformation of the used form of aripiprazole into form X.

6. Process according to claim 5, wherein the solvent is 2-propanol and the preferred temperature range is about 40 °C to about 60 °C.

## Patentansprüche

1. Verfahren zur Herstellung von Form X von Aripiprazol, **gekennzeichnet durch** ein Röntgenstrahlen-Pulver Diffraktogramm mit Peaks bei 10,0, 11,6, 15,7, 16,3, 18,5, 20,4, 21,8, 22,2 und 23,3 Grad 2θ (2-theta), umfassend die Schritte
a) Auflösen von Aripiprazol in 2-Propanol **durch** Erwärmen
b) langsames Abkühlen der Lösung auf Raumtemperatur oder darunter, um Kristallisation zu bewirken
c) gegebenenfalls Isolieren der kristallinen Form X von Aripiprazol.

2. Verfahren nach Anspruch 1, wobei die in Schritt a) verwendete Form Aripiprazol-Ethanol-Hemisolvat und/oder Aripiprazol-Methanol-Solvat und/oder Aripiprazol-Hydrat ist.

3. Verfahren zur Herstellung von Form X von Aripiprazol nach Anspruch 1 oder Anspruch 2, wobei in Schritt b) Impfkristalle von Form von X von Aripiprazol zugegeben werden.

4. Verfahren nach Anspruch 3, wobei das Lösungsmittel 2-Propanol ist und der bevorzugte Temperaturbereich etwa 40 °C bis etwa 70 °C beträgt.

5. Verfahren zur Herstellung von Form X von Aripiprazol, **gekennzeichnet durch** ein Röntgenstrahl-Pulver Diffraktogramm mit Peaks bei 10,0, 11,6, 15,7, 16,3, 18,5, 20,4, 21,8, 22,2 und 23,3 Grad 2θ (2-theta), **dadurch** gekennzeichnet, dass eine Suspension von Aripiprazol in einem Lösungsmittel, ausgewählt aus 2-Propanol, 1-Butanol oder 1-Propanol mit Kristallen von Form X angeimpft wird und die Suspension bei einer geeigneten Temperatur gerührt wird, um die Umwandlung von der verwendeten Form von Aripiprazol in Form X zu bewirken.

6. Verfahren nach Anspruch 5, wobei das Lösungsmittel 2-Propanol ist und der bevorzugte Temperaturbereich etwa 40 °C bis etwa 60 °C beträgt.

## Revendications

1. Procédé de préparation de la forme X de l'aripiprazole **caractérisé par** un diagramme de diffraction des rayons X sur poudre avec des pics à 10,0, 11,6, 15,7, 16,3, 18,5, 20,4, 21,8, 22,2 et 23,3 degrés 2θ (2-thêta), comprenant les étapes de :
a) dissolution de l'aripiprazole dans du 2-propanol par chauffage
b) refroidissement lent de la solution à la température ambiante ou au-dessous pour réaliser une cristallisation
c) éventuellement, isolement de la forme X cristalline de l'aripiprazole.

2. Procédé selon la revendication 1, dans lequel la forme utilisée à l'étape a) est l'hémisolvate d'aripiprazole avec l'éthanol et/ou le solvate d'aripiprazole avec le méthanol et/ou l'hydrate d'aripiprazole.

3. Procédé de préparation de la forme X de l'aripiprazole selon la revendication 1 ou la revendication 2, dans lequel à l'étape b) des cristaux d'ensemencement de la forme X de l'aripiprazole sont ajoutés.

4. Procédé selon la revendication 3, dans lequel le solvant est le 2-propanol et la plage de température préférée est d'environ 40°C à environ 70°C.

5. Procédé de préparation de la forme X de l'aripiprazole **caractérisé par** un diagramme de diffraction des rayons X sur poudre avec des pics à 10,0, 11,6, 15,7, 16,3, 18,5, 20,4, 21,8, 22,2 et 23,3 degrés 2θ (2-thêta), **caractérisé en ce qu'**une suspension d'aripiprazole dans un solvant choisi parmi le 2-propanol, le 1-butanol ou le 1-propanol est ensemencée avec des cristaux de la forme X et la suspension est agitée à une température appropriée afin d'effectuer une transformation de la forme utilisée d'aripiprazole en forme X.

6. Procédé selon la revendication 5, dans lequel le solvant est le 2-propanol et la plage de température préférée est d'environ 40°C à environ 60°C.
